# EUROPEAN PATENT APPLICATION

(11) **EP 2 756 801 A1**
(43) Date of publication of application: **23.07.2014**
(21) Application number: 12832314.4
(22) Date of filing: 11.09.2012
(51) Int. Cl.: A61B 6/00, G06T 1/00, G06T 5/00, H04N 1/40

(54) **IMAGE PROCESSING DEVICE**

(30) Priority: 14.09.2011 JP 2011200061
(71) Applicant: FUJIFILM Corporation, Tokyo 106-8620 (JP)
(72) Inventor: FUKUDA, Wataru, Ashigarakami-gun Kanagawa 258-8538 (JP)
(74) Representative: Klunker . Schmitt-Nilson . Hirsch
(86) International application number: PCT/JP2012/073167
(87) International publication number: WO 2013/039054

(57) **Abstract**

*Provided is an image processing device for blacking out a scattered ray region without losing the imaging object information in the scattered ray region of a radiograph. An image processing device (56) for executing a concentration correction process on an obtained radiograph by using a recording plate (52) capable of accumulating radiographic information, wherein the radiograph contains a valid image region (72) corresponding to a region in which the recording plate (52) is present, and an invalid image region (74) corresponding to the region in which the recording plate (52) is not present. Furthermore, the image processing device (56) is provided with: a region-to-be-corrected identi fication unit (82) for identifying a pre-set range of regions (94) within the valid image region (72) that are adjacent to the invalid image region (74) as to-be-corrected regions (90); and concentration correction units (84, 120) for correcting the concentration level of pixels in the to-be-corrected regions (90) by removing the concentration level trend in the to-be-corrected regions (90) that become present when moving from the valid image region (72) side toward the invalid image region (74) side.*

## Description

### Technical Field

The present invention relates to an image processing apparatus (image processing device) for performing a blackening process on a radiographic image.

### Background Art

Imaging plates (hereinafter referred to as "IP") have shapes with round corners. Therefore, a radiographic image, which has been captured using an IP, has white spots on corners thereof that are free of the IP. Even the side edges of the radiographic image may have white spots due to wobbling movement caused at times that the IP reading device is carried. It is further known that image portions, which do not have white spots but are positioned adjacent to white spots, have regions that are significantly affected by scattering rays. The white spots are highly luminous, and the regions that are significantly affected by scattering rays, referred to as scattered-ray regions, are relatively highly luminous. Therefore, the white spots and the scattered-ray regions tend to bring about a reduction in visibility of the radiographic image, and tend to cause strain on the eyes of the observer.

Japanese Laid-Open Patent Publication No. 2004-283281 discloses a technique for automatically blackening white spots and scattered-ray regions on side edges of a photographic image that is captured using an IP.

### Summary of Invention

However, if a scattered-ray region of a radiographic image is simply blackened out, then subject information represented by the scattered-ray region is lost. Conversely, if a scattered-ray region is not blackened out, the visibility of the radiographic image is lowered as a result of the scattered-ray region being relatively highly luminous.

The present invention has been made in view of the aforementioned problems. It is an object of the present invention to provide an image processing apparatus for blackening out a scattered-ray region without causing a loss in subject information represented by the scattered-ray region.

To achieve the above object, there is provided in accordance with the present invention an image processing apparatus for performing a density correcting process on a radiographic image acquired using a recording plate that is capable of storing radiation information, wherein the radiographic image includes a valid image region corresponding to a region in which the recording plate is present, and an invalid image region corresponding to a region in which the recording plate is not present, the image processing apparatus comprising a correction target region identifying unit for identifying a region in a predetermined range of the valid image region, which is adjacent to the invalid image region, as a correction target region, and a density correction unit for removing a trend of density values of the correction target region, which is present from the valid image region toward the invalid image region, thereby correcting the density values of pixels in the correction target region.

To achieve the aforementioned object, there also is provided in accordance with the present invention an image processing apparatus for performing a density correcting process on a radiographic image acquired using a solid-state detector, wherein the radiographic image includes a valid image region corresponding to a region that is irradiated with radiation, and an invalid image region corresponding to a region that is not irradiated with radiation, the image processing apparatus comprising a correction target region identifying unit for identifying a region in a predetermined range of the valid image region, which is adjacent to the invalid image region, as a correction target region, and a density correction unit for removing a trend of density values of the correction target region, which is present from the valid image region toward the invalid image region, thereby correcting the density values of pixels in the correction target region.

The density correction unit may correct the density values of pixels in the correction target region by increasing density values to be added to pixels in the correction target region as the pixels are closer to the invalid image region.

Within the valid image region, a region other than the correction target region may be regarded as a region of interest, and the density correction unit may include a map calculator for calculating an additive quantity map representing additive quantities to be added to the density values of a plurality of pixels in the correction target region by calculating differences between the density values of a plurality of reference pixels serving as a reference for calculating the additive quantity map in the region of interest and the density values of the pixels in the correction target region, a low-pass filtering processor for performing a low-pass filtering process on the additive quantity map, and a density value adder for adding the additive quantity map, which has been processed by the low-pass filtering process, to the density values of the pixels in the correction target region, so that the density correction unit removes the trend of density values of the correction target region.

Within the valid image region, a region other than the correction target region may be regarded as a region of interest, and the density correction unit may include a low-pass filtering processor for performing a low-pass filtering process on the density values of a plurality of pixels in the correction target region, a map calculator for calculating an additive quantity map representing additive quantities to be added to the density values of the pixels in the correction target region by calculating differences between the density values of a plurality of reference pixels serving as a reference for calculating the additive quantity map in the region of interest and the density values of the pixels in the correction target region, which have been processed by the low-pass filtering process, and a density value adder for adding the additive quantity map to the density values of the pixels in the correction target region, so that the density correction unit removes the trend of density values of the correction target region.

The map calculator may calculate the additive quantity map by calculating differences between the density values of the pixels in the correction target region and the density values of the reference pixels that are shortest in distance from the pixels in the correction target region.

Within the valid image region, a region other than the correction target region may be regarded as a region of interest, and the density correction unit may include a low-pass filtering processor for performing a low-pass filtering process on the density values of a plurality of pixels in the correction target region, a differential density value calculator for calculating differential density values of the pixels in the correction target region by calculating differences between the density values of the pixels in the correction target region and the density values of the pixels in the correction target region, which have been processed by the low-pass filtering process, and an adder for adding the density values of a plurality of reference pixels serving as a reference in the region of interest to the differential density values of the pixels in the correction target region, so that the density correction unit corrects the density values of the pixels in the correction target region.

The adder may add the pixels in the correction target region and the reference pixels that are shortest in distance from the pixels in the correction target region.

A boundary line between the correction target region and the region of interest and a boundary line between the valid image region and the invalid image region may lie parallel to each other.

The reference pixels may comprise pixels in the region of interest that are adjacent to the pixels in the correction target region.

The correction target region identifying unit may identify, as the correction target region, a region in the valid image region that lies within a constant distance from a boundary line between the valid image region and the invalid image region.

The correction target region identifying unit may identify, as the correction target region, the invalid image region together with the region in the predetermined range of the valid image region that is adjacent to the invalid image region.

The density correction unit may blacken out the invalid image region by replacing the density values of pixels in the invalid image region with a predetermined density value.

According to the present invention, the trend of density values of the correction target region, which is present from the valid image region toward the invalid image region of the radiographic image, can be removed, thereby correcting and blackening out the density values of pixels that lie within the correction target region. Consequently, a scattered-ray region can be blackened out while retaining subject information.

### Brief Description of Drawings

FIG. 1 is a perspective view of a mammographic apparatus, which serves as an example of a radiographic image capturing apparatus;
FIG. 2 is an enlarged fragmentary side elevational view of the mammographic apparatus shown in FIG. 1;
FIG. 3 is an electric block diagram of an image processing system for reading and processing a radiographic image stored in an IP;
FIG. 4 is a diagram showing a corner of a radiographic image read by an image reader;
FIG. 5 is a block diagram of an image processing apparatus shown in FIG. 3;
FIG. 6 is a diagram illustrating a specified correction target region;
FIG. 7 is a flowchart of an operation sequence of the image processing apparatus shown in FIG. 3;
FIG. 8 is a diagram illustrating a process of calculating an additive quantity map;
FIG. 9 is a diagram showing a relationship between the density value of each pixel on a normal line A shown in FIG. 8 and the additive quantity to be added to each pixel in a correction target region on the normal line A;
FIG. 10 is a diagram showing an image of an additive quantity map calculated from the radiographic image shown in FIG. 4, which is read by the image reader and processed by a low-pass filtering process;
FIG. 11 is a diagram showing the density value of each pixel of the correction target region on the normal line A shown in FIG. 9, at a time that the additive quantity map is added to density values of a plurality of pixels of the correction target region;
FIG. 12 is a diagram showing a radiographic image, which is generated after the additive quantity map processed by the low-pass filtering process is added to density values of the pixels of the correction target region, for a case in which the radiographic image shown in FIG. 4 is read by the image reader;
FIG. 13 is a block diagram of a density correction unit according to a fourth modification; and
FIG. 14 is a diagram showing a radiographic image generated by blackening out an invalid image region by correcting density values of a region using an additive quantity map, and thereafter replacing a plurality of pixel values in the invalid image region with a predetermined density value.

### Description of Embodiments

An image processing apparatus according to a preferred embodiment of the present invention will be described in detail below with reference to the accompanying drawings.

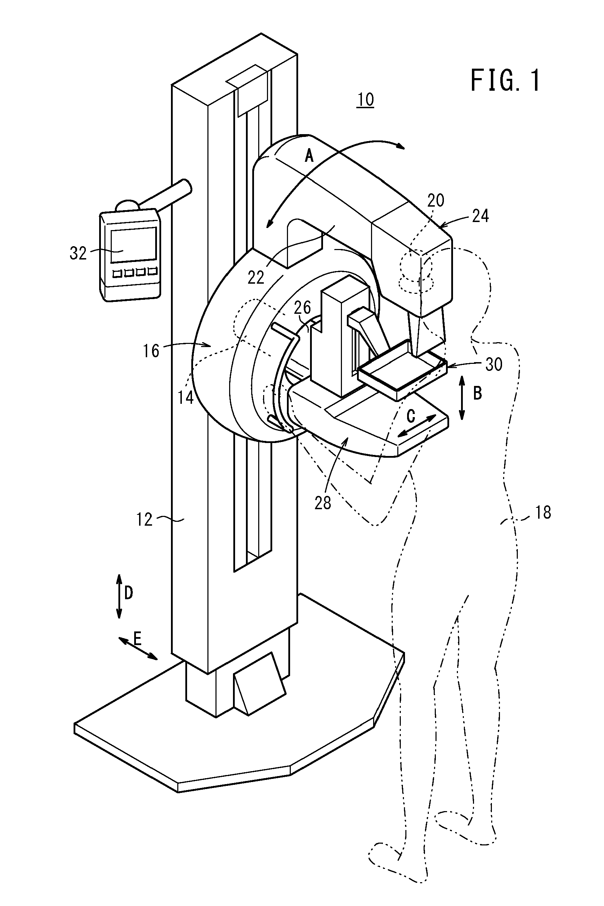

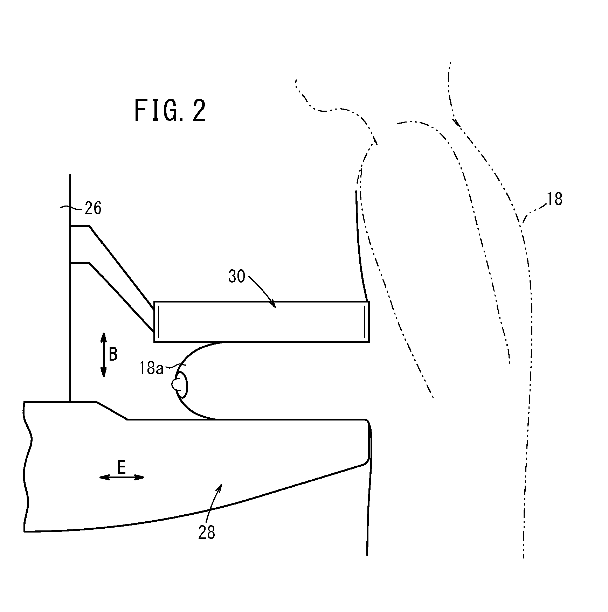

FIG. 1 is a perspective view of a mammographic apparatus 10, which serves as an example of a radiographic image capturing apparatus. FIG. 2 is an enlarged fragmentary side elevational view of the mammographic apparatus 10. The mammographic apparatus 10 includes an upstanding base 12, a main apparatus unit 16 fixed to a swing shaft 14 disposed substantially centrally on the base 12, a radiation source housing unit 24 housing therein a radiation source 20 such as a molybdenum tube, a tungsten tube, a rhodium tube, or the like, for applying radiation (e.g., X-rays) to an object to be examined of a subject 18, and which is fixed to an end of an arm 22 of the main apparatus unit 16, an image capturing base 28 which is fixed to a bracket 26 of the main apparatus unit 16 and housing therein a solid-state detector, not shown, for detecting radiation that has passed through a breast 18a and acquiring radiographic image information, and a compression plate 30 mounted on the bracket 26 for compressing and holding the breast 18a against the image capturing base 28.

The solid-state detector, which comprises an IP (stimulable light emission plate), acquires a radiographic image of the subject by storing radiation that has passed through the breast 18a. The IP stores radiation that has passed through the breast 18a, and upon being irradiated with stimulating light, is stimulated to emit light having an intensity that depends on the dose of stored radiation. The IP includes a stimulable phosphor layer disposed on a substrate. A display operation unit 32 is mounted on the base 12 for displaying image capturing information, such as an image capturing direction, etc., and ID information of the subject 18. The display operation unit 32 also enables such items of information to be set as necessary.

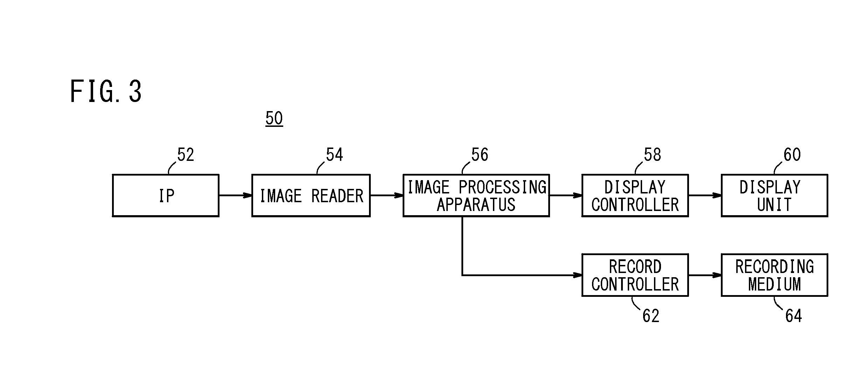

An image processing system 50, as shown in FIG. 3, will be described below. FIG. 3 is an electric block diagram of an image processing system 50 for reading and processing a radiographic image that is stored in an IP 52. The image processing system 50 includes the IP 52, an image reader 54, an image processing apparatus 56, a display controller 58, a display unit 60, a record controller 62, and a recording medium 64.

The image reader 54 reads image information of the subject that has been detected by the IP 52 (recording plate), converts the read radiographic image of the subject into digital signals, and supplies the digital signals to the image processing apparatus 56. More specifically, the image reader 54 includes a light source for emitting stimulating light, a scanner for sweeping the stimulating light, a photomultiplier, an amplifier, and an A/D converter (not shown). The scanner scans the surface of the IP 52 with stimulating light. The scanner scans the surface of the IP 52, whereupon stimulating light is applied to the IP 52, and the region of the IP 52 that is irradiated with stimulating light emits an amount of light that depends on the stored radiographic image of the subject. The photomultiplier converts the emitted light into electric signals, and the amplifier amplifies the electric signals. The A/D converter converts the amplified electric signals into digital signals, thereby producing a radiographic image represented by the digital signals.

The image processing apparatus 56 carries out predetermined image processing, to be described later, on the radiographic image read by the image reader 54, and supplies the processed radiographic image to at least one of the display controller 58 and the record controller 62.

The display controller 58 displays the radiographic image on the display unit 60, which may comprise a liquid crystal display panel or the like. The record controller 62 records the radiographic image in the recording medium 64, which may comprise a flash memory, a hard disk, or the like.

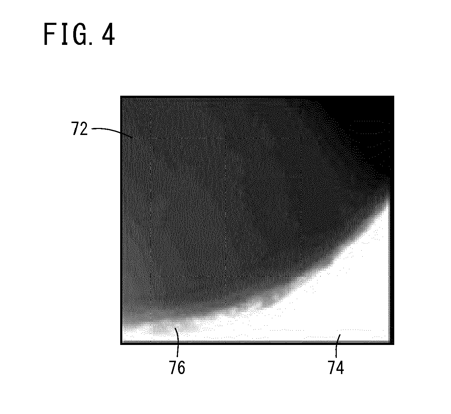

FIG. 4 is a diagram showing a corner of a radiographic image read by the image reader 54. As shown in FIG. 4, the radiographic image includes a valid image region 72 that corresponds to a region in which the IP 52 is present, and an invalid image region 74 that corresponds to a region in which the IP 52 is not present. As described above, since the IP 52 has a shape with rounded corners, the IP 52 is incapable of storing radiation around the corners even if the IP 52 is irradiated with radiation. Therefore, the invalid image region 74 is highly luminous and is observed as a white spot. In addition, the valid image region 72 includes a scattered-ray region 76 that corresponds to an edge of the IP 52. Since the edge of the IP 52 scatters radiation, the scattered-ray region 76 is observed as a blurred image. The density value of each pixel of the radiographic image varies depending on the dose of radiation applied to the IP 52. As the applied dose of radiation becomes smaller, the density value also becomes smaller. The density value is low in the invalid image region 74 and varies within the scattered-ray region 76. The density value becomes smaller toward the edge of the IP 52. In corners of the radiographic image, a trend of density values is present from the valid image region 72 toward the invalid image region 74.

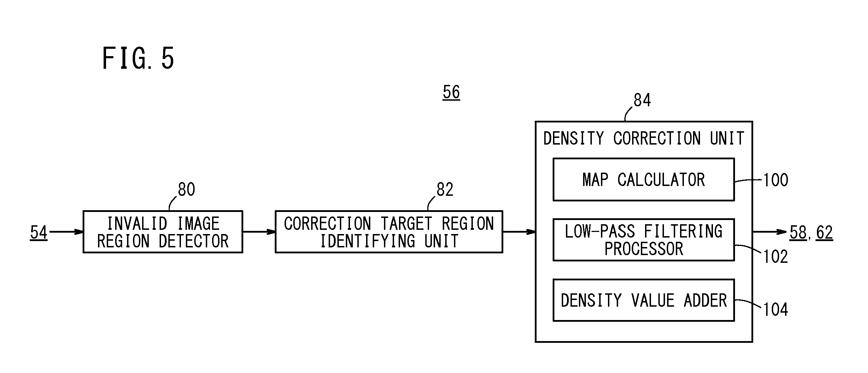

FIG. 5 is a block diagram of the image processing apparatus 56. The image processing apparatus 56 includes an invalid image region detector 80, a correction target region identifying unit 82, and a density correction unit 84.

The invalid image region detector 80 detects the invalid image region 74 of the radiographic image that is sent from the image reader 54. The invalid image region detector 80 may detect the invalid image region 74 by separating a background region and a profile region based on clustering according to a K-means algorithm and regarding the background region as the invalid image region 74, as disclosed in Japanese Patent No. 2831892, for example. Alternatively, as disclosed in Japanese Laid-Open Patent Publication No. 2004-283281, the invalid image region detector 80 may detect the invalid image region 74 by detecting an edge. Further, alternatively, the invalid image region detector 80 may detect the invalid image region 74 by comparing the density value of each pixel of the radiographic image with a threshold value, and regarding a group of pixels the density values of which are lower than the threshold value as the invalid image region 74. According to the present embodiment, the invalid image region detector 80 detects the invalid image region 74 by comparing the density value of each pixel of the radiographic image with a threshold value. The invalid image region detector 80 supplies the radiographic image that was sent thereto to the correction target region identifying unit 82, and also supplies information representing the detected invalid image region 74.

Based on the detected invalid image region 74, the correction target region identifying unit 82 identifies a region to be corrected, i.e., a correction target region, of the radiographic image where density values of the pixels are to be corrected. The correction target region identifying unit 82 identifies the invalid image region 74 together with a region in the valid image region 72, which lies within a predetermined range adjacent to the invalid image region 74, as a correction target region. The correction target region identifying unit 82 supplies the radiographic image that was sent thereto to the density correction unit 84, and also supplies information representing the specified correction target region and the invalid image region 74.

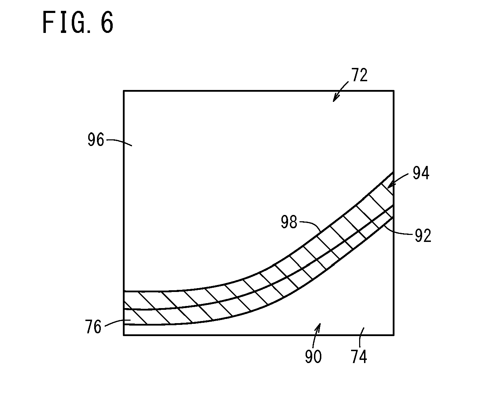

FIG. 6 is a diagram illustrating a specified correction target region 90. The region in the valid image region 72, which lies within the predetermined range adjacent to the invalid image region 74, may refer to a region (shown in hatching) 94 that lies within the valid image region 72 and which falls at a constant distance from a boundary line 92 between the valid image region 72 and the invalid image region 74. The length of the constant distance is determined such that the region 94 includes the scattered-ray region 76 in its entirety.

In the valid image region 72, a region other than the correction target region 90 (region 94) is regarded as a region of interest 96. A boundary line 98 between the region of interest 96 and the correction target region 90, i.e., a boundary line between the region of interest 96 and the region 94, lies parallel to the boundary line 92. The boundary line 92 is made up of a plurality of pixels in the valid image region 72, which are adjacent to the pixels of the invalid image region 74. The boundary line 98 is made up of a plurality of pixels in the region of interest 96, which are adjacent to the pixels of the region 94. The pixels in the region of interest 96 that make up the boundary line 98, i.e., the pixels on the boundary line 98, are referred to as reference pixels.

The density correction unit 84 removes the trend of pixel values of the pixels in the correction target region 90, which is present from the valid image region 72 toward the invalid image region 74, thereby correcting (blackening) the density values of the pixels in the correction target region 90 so as to blacken out the correction target region 90.

The density correction unit 84 includes a map calculator 100, a low-pass filtering processor 102, and a density value adder 104. The map calculator 100 calculates an additive quantity map by calculating differences between the density values of the reference pixels and the density values of the pixels in the correction target region 90. The additive quantity map refers to a map representing additive quantities (density values) to be added to the density values of the pixels in the correction target region 90. The map calculator 100 may also calculate an additive quantity map by calculating differences between the density values of the pixels in the correction target region 90 and the density values of the reference pixels that are shortest in distance from the pixels in the correction target region 90.

The low-pass filtering processor 102 performs a low-pass filtering process on the calculated additive quantity map to thereby remove high-frequency components from the additive quantity map. The density correction unit 84 adds the additive quantity map processed by the low-pass filtering process to the density values of the pixels in the correction target region 90, thereby correcting the density values of the pixels in the correction target region 90.

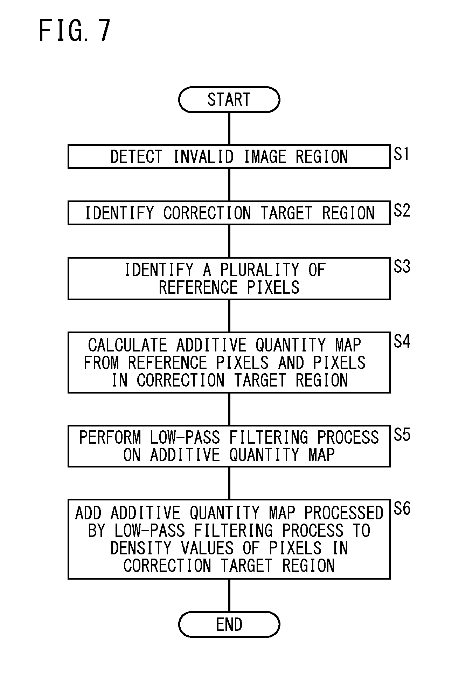

Operations of the image processing apparatus 56 will be described below with reference to the flowchart shown in FIG. 7. First, if a radiographic image is sent from the image reader 54 to the image processing apparatus 56, the invalid image region detector 80 detects an invalid image region 74 in the radiographic image that was sent to the image processing apparatus 56 (step S1).

Next, based on the detected invalid image region 74, the correction target region identifying unit 82 identifies a correction target region 90 where the density values of pixels are to be corrected in the radiographic image (step S2). The correction target region identifying unit 82 then identifies, as a correction target region 90, a region 94 that lies within the invalid image region 74, and a predetermined range in the valid image region 72 that lies adjacent to the invalid image region 74 (see FIG. 6). Due to the fact that the scattered-ray region 76, which is difficult to detect accurately, is to be included within the correction target region 90, the correction target region identifying unit 82 identifies not only the scattered-ray region 76 and the invalid image region 74 as the correction target region 90, but also identifies, as the correction target region 90, the region 94 including the scattered-ray region 76 and the invalid image region 74.

Next, the map calculator 100 of the density correction unit 84 identifies a plurality of reference pixels from the pixels of the region of interest 96 of the radiographic image (step S3). More specifically, the map calculator 100 identifies, as reference pixels, a plurality of pixels in the region of interest 96, which are present on the boundary line 98 between the region of interest 96 and the correction target region 90.

Thereafter, the map calculator 100 of the density correction unit 84 calculates an additive quantity map representing additive quantities to be added to the density values of the pixels in the correction target region 90, by calculating differences between the density values of the pixels in the correction target region 90 and the density values of the reference pixels that are shortest in distance from the pixels in the correction target region 90 (step S4). The map calculator 100 calculates the differences between the density values of the pixels in the correction target region 90 and the density values of the reference pixels that are shortest in distance from the pixels in the correction target region 90, by subtracting the density values of the pixels in the correction target region 90 from the density values of the reference pixels.

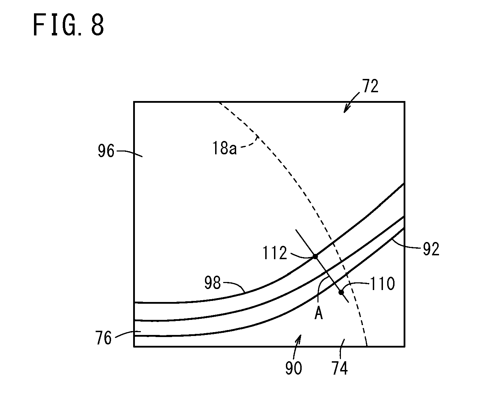

FIG. 8 is a diagram illustrating a process of calculating an additive quantity map. Since the map calculator 100 calculates differences between the density values of the pixels in the correction target region 90 and the density values of the reference pixels that are shortest in distance from the pixels in the correction target region 90, the pixels in the correction target region 90 and the reference pixels are both present on the same normal lines to the boundary line 98. For example, a pixel 110 in the correction target region 90 and a reference pixel 112 shortest in distance from the pixel 110 both are present on a normal line A, which is normal to the boundary line 98 on the reference pixel 112. The map calculator 100 calculates an additive quantity map by calculating, on each line normal to the boundary line 98, the difference between the density value of the reference pixel and the density value of each pixel in the correction target region 90. In FIG. 8, the breast 18a is illustrated as an object whose image is to be captured.

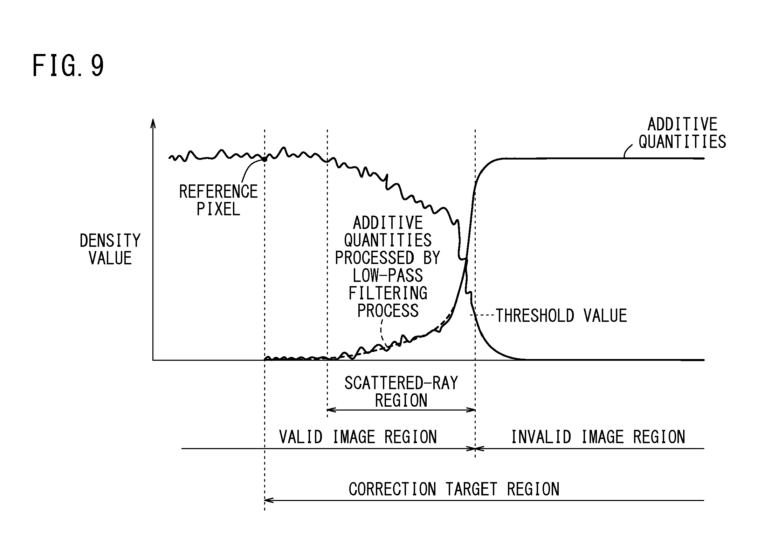

FIG. 9 is a diagram showing a relationship between the density value of each pixel on a normal line A shown in FIG. 8 and the additive quantity to be added to each pixel in the correction target region 90 on the normal line A. As shown in FIG. 9, a region in which the pixel density values are lower than the threshold value referred to above is indicated as an invalid image region 74, and a region in which the pixel density values are higher than the threshold value referred to above is indicated as a valid image region 72. The invalid image region 74 and a region 94 including the scattered-ray region 76 are indicated as a correction target region 90. It can be seen from the figure that the scattered-ray region 76 has pixel values therein that vary significantly.

As shown in FIG. 9, the density value of each pixel in the correction target region 90 is subtracted from the density value of a reference pixel on a normal line A, whereby an additive quantity, which is to be added to each pixel in the correction target region 90 on the normal line A, is determined.

Next, the low-pass filtering processor 102 of the density correction unit 84 performs a low-pass filtering process on the additive quantity map that was calculated in step S4, to thereby remove high-frequency components from the additive quantity map (step S5). Upon the low-pass filtering process being performed on the additive quantity map, as shown in FIG. 9, a curve (indicated by the broken line in FIG. 9), which is represented by the additive quantities to be added to the pixels in the correction target region 90, is smoothed. In the correction target region 90, pixels with lower density values are combined with greater additive quantities, which are added thereto. In other words, pixels that are closer to the invalid image region 74 are combined with greater additive quantities, which are added thereto, and the pixels of the invalid image region 74 are combined with the greatest additive quantity, which is added thereto.

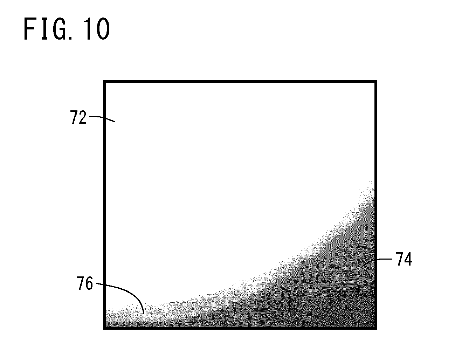

FIG. 10 is a diagram showing an image of an additive quantity map, which is calculated from the radiographic image shown in FIG. 4, and which is read by the image reader 54 and processed by a low-pass filtering process. As shown in FIG. 10, image regions corresponding to the invalid image region 74 and the scattered-ray region 76 are blackened out, and an image region corresponding to the valid image region 72 other than the scattered-ray region 76 is observed as a white spot.

Next, the density value adder 104 adds the additive quantity map to the density values of the pixels in the correction target region 90 of the radiographic image that is read by the image reader 54 (step S6).

In step S6, rather than the additive quantity map calculated in step S4, the density value adder 104 adds the additive quantity map that was processed by the low-pass filtering process in step S5 to the density values of the pixels in the correction target region 90 for the following reasons. Namely, the high-frequency components in the correction target region 90 represent subject information. If an additive quantity map that was not processed by the low-pass filtering process is added to the density values of the pixels in the correction target region 90, then subject information in the correction target region 90 is lost. On the other hand, if the additive quantity map that was processed by the low-pass filtering process is added to the density values of the pixels in the correction target region 90, the correction target region 90 is blackened out while retaining the subject information (high-frequency components) in the correction target region 90.

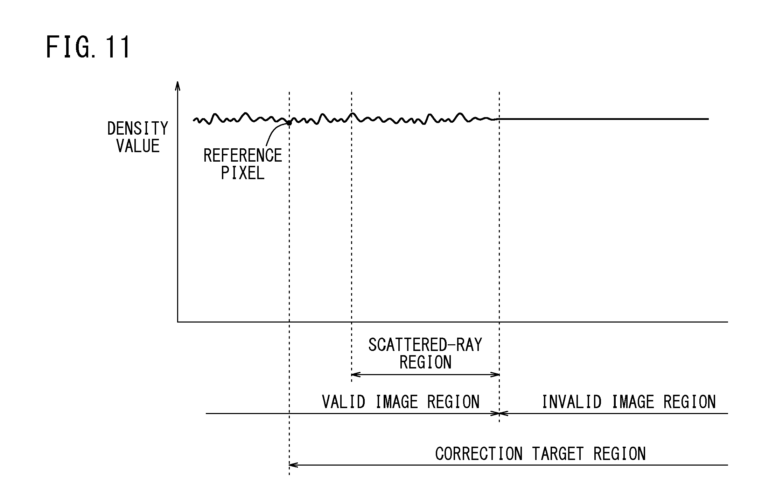

FIG. 11 is a diagram showing the density value of each pixel in the correction target region 90 on the normal line A shown in FIG. 9, at a time that the additive quantity map is added to the density values of the pixels of the correction target region. As shown in FIG. 11, the correction target region 90 is blackened out, i.e., density values of the pixels in the correction target region 90 are increased, without removing subject information (high-frequency components) in the scattered-ray region 76. Similarly, the invalid image region 74 is blackened out.

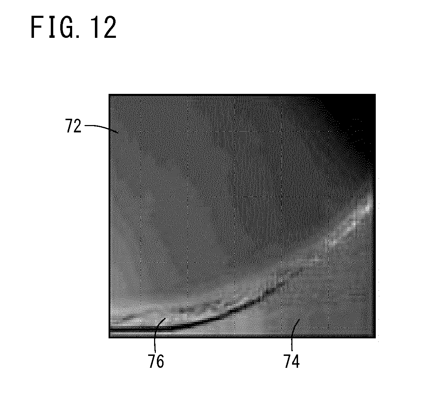

FIG. 12 is a diagram showing a radiographic image generated after the additive quantity map, which was processed by the low-pass filtering process, is added to the density values of the pixels in the correction target region 90, for a case in which the radiographic image shown in FIG. 4 is read by the image reader 54. As shown in FIG. 12, the invalid image region 74 and the scattered-ray region 76 are blackened out substantially uniformly.

As described above, an additive quantity map is calculated by subtracting the density values of a plurality of pixels in the correction target region 90 from the density values of a plurality of reference pixels. Further, after the additive quantity map has been processed by the low-pass filtering process, the processed additive quantity map is added to the density values of the pixels in the correction target region 90. Therefore, a trend of the density values of the pixels in the correction target region 90, which is present from the valid image region 72 toward the invalid image region 74, is removed, and the correction target region 90 is blackened out. In other words, the scattered-ray region 76 is blackened out while retaining subject information (high-frequency components) of the scattered-ray region 76, and the invalid image region 74 also is blackened out. Although it is difficult to accurately detect the scattered-ray region 76 from the radiographic image, the scattered-ray region 76 is blackened out without removing the subject information of the scattered-ray region 76, regardless of the difficulty in accurately detecting the scattered-ray region 76 according to the present embodiment. Since the additive quantities that are added to the density values of pixels in the valid image region 72, which is not the scattered-ray region 76, of the correction target region 90 are extremely small, even if the region 94 is greater than the scattered-ray region 76, the density values of the valid image region 72, which is not the scattered-ray region 76, are not significantly affected by the density correction, but remain essentially uncorrected. In this manner, no subject information is lost.

Conversely, if the density values of the pixels in the correction target region 90 are corrected by being replaced with a predetermined value, then the subject information in the valid image region 72 is lost. According to the present embodiment, such a problem does not occur.

According to the above embodiment, since greater additive quantities are added to the pixels in the correction target region 90, the density values of which are lower than the density value of the reference pixels, the correction target region 90 is blackened out substantially uniformly while retaining subject information therein.

In the case that a radiographic image of the breast 18a is captured, a line tangential to an edge of the breast 18a in the scattered-ray region 76 is close to the normal line A, which is normal to the boundary line 98, as shown in FIG. 8. Consequently, calculation of the additive quantity map, which is calculated by determining the difference between the density value of a reference pixel and the density value of a pixel in the correction target region 90 on respective normal lines that are normal to the boundary line 98 and then adding the calculated additive quantity map, makes it possible to blacken out the scattered-ray region 76 while sufficiently retaining subject information therein.

The above embodiment can be modified in the following ways.

### (Modification 1)

In the above-described embodiment, the map calculator 100 calculates an additive quantity map by subtracting the density values of a plurality of pixels in the correction target region 90 from the density values of a plurality of reference pixels (step S4), and thereafter, the low-pass filtering processor 102 performs a low-pass filtering process on the calculated additive quantity map (step S5). However, the low-pass filtering processor 102 may perform a low-pass filtering process on the density values of a plurality of pixels in the correction target region 90, and thereafter, the map calculator 100 may determine an additive quantity map by subtracting the density values of the pixels in the correction target region 90, which have been processed by the low-pass filtering process, from the density values of a plurality of reference pixels. According to this modification, although the low-pass filtering process is not performed on the additive quantity map, the same additive quantity map as that generated in the above-described embodiment is obtained, the trend of the density values of the pixels in the correction target region 90, which is present from the valid image region 72 toward the invalid image region 74, is removed, and the correction target region 90 is blackened out.

### (Modification 2)

In the above-described embodiment, an additive quantity map is calculated by calculating the difference between the density values of reference pixels and the density values of pixels in the correction target region 90, both of which are on the same normal lines. However, an additive quantity map may be calculated by calculating the difference between the density values of reference pixels and the density values of pixels in the correction target region 90, which are not on the same normal lines, but are on the same straight lines, for example. For example, an additive quantity map may be calculated by calculating the difference between the density values of reference pixels and the density values of pixels in the correction target region 90, which are on the same straight lines that lie parallel to a y-axis direction (vertical direction of the radiographic image) or an x-axis direction (horizontal direction of the radiographic image).

### (Modification 3)

In the above-described embodiment, pixels on the boundary line 98 between the region of interest 96 and the correction target region 90 are used as reference pixels. However, the reference pixels may be pixels that lie within the region of interest 96.

### (Modification 4)

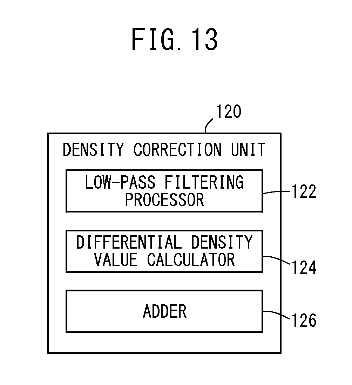

According to Modification 4, the density correction unit 84 of the image processing apparatus 56 may be replaced with a density correction unit 120 shown in FIG. 13. Further, according to Modification 4, the density correction unit 120 includes a low-pass filtering processor 122, a differential density value calculator 124, and an adder 126. The low-pass filtering processor 122 performs a low-pass filtering process on the density values of a plurality of pixels in the correction target region 90 in order to remove high-frequency components. The differential density value calculator 124 calculates differences between the density values of the pixels in the correction target region 90 and the density values of the pixels in the correction target region 90, which have been processed by the low-pass filtering process, thereby calculating differential density values of the pixels in the correction target region 90. The adder 126 adds the density values of a plurality of reference pixels to the calculated differential density values of the pixels in the correction target region 90. The adder 126 adds the reference pixels and the pixels in the correction target region 90, which are present on the same straight lines, e.g., normal lines. According to Modification 4, the trend of the density values of the pixels in the correction target region 90, which is present from the valid image region 72 toward the invalid image region 74, is removed, and the correction target region 90 is blackened out.

### (Modification 5)

In step S6 shown in FIG. 7, after the density correction unit 84 has added the additive quantity map to the density values of the pixels in the correction target region 90, thereby blackening out the pixels in the correction target region 90, the density correction unit 84 may blacken out the image of the invalid image region 74. For example, the density correction unit 84 may blacken out the invalid image region 74 by replacing the density values of the pixels in the invalid image region 74 with a predetermined density value.

In the above-described embodiment, in step S2 of FIG. 7, the correction target region identifying unit 82 identifies the region 94 and the invalid image region 74 as a correction target region 90. However, according to the present modification, the correction target region identifying unit 82 may identify only the region 94 as a correction target region 90. In this case, in step S6, the density values of the region 94 identified as a correction target region 90 are corrected by the additive quantity map, and the correction target region 90 is blackened out while retaining the subject information therein. Since the density values of the invalid image region 74 are not corrected, the invalid image region 74 is blackened out by replacing the density values of the pixels in the invalid image region 74 with a predetermined density value.

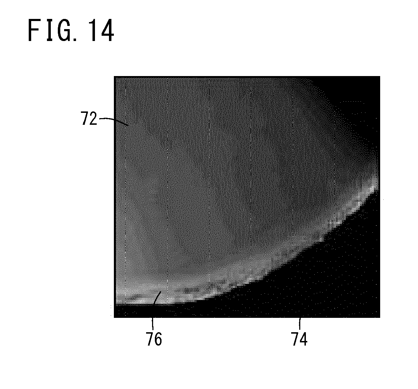

FIG. 14 is a diagram showing a radiographic image, which is generated upon blackening out the invalid image region 74 by correcting the density values of the region 94 using an additive quantity map, and thereafter replacing a plurality of pixel values in the invalid image region 74 with a predetermined density value.

### (Modification 6)

In the above embodiment, the entire region of the IP is irradiated with radiation. However, the irradiation field may be reduced such that only a portion of the entire region of the IP is irradiated with radiation. In this case, while the region of the IP that is irradiated with radiation is able to produce image information concerning the subject, the region of the IP that is not irradiated with radiation is unable to produce image information of the subject, but instead produces a white spot.

Even if the irradiation field is reduced while capturing the radiographic image, a scattered-ray region in which the radiation is scattered is produced between the region that is irradiated with radiation and the region that is not irradiated with radiation. The same processing sequence as with the above embodiment then is carried out in order to blacken out the scattered-ray region while retaining the subject information of the scattered-ray region, and also to blacken out the region that is not irradiated with radiation.

More specifically, according to Modification 6, among the radiographic image read from the IP 52, which has been captured in the reduced irradiation field, a region corresponding to the region irradiated with radiation is regarded as the valid image region 72 in the above-described embodiment, a region corresponding to the region that is not irradiated with radiation is regarded as the invalid image region 74 in the above-described embodiment, and a scattered-ray region, which is produced between the valid image region 72 and the invalid image region 74, is regarded as the scattered-ray region 76 in the above-described embodiment. The operational sequence according to the flowchart shown in FIG. 7 is performed in order to blacken out the correction target region 90, which includes the scattered-ray region 76.

In Modification 6, the solid-state detector is not limited to the IP 52, but may comprise a DR (digital radiography) panel such as an electronic cassette (FPD) or the like, which is capable of detecting radiation electrically.

### (Modification 7)

Modifications 1 through 6 described above may be combined in any way, insofar as no inconsistencies occur in the resultant combinations.

The present invention has been described above in connection with a given preferred embodiment. However, the technical scope of the present invention is not limited to the description of the above embodiment. It will be obvious to those skilled in the art that various changes or improvements may be made to the above embodiment. It is apparent from the description of the scope of the claims for patent that such changes or modifications fall within the technical scope of the present invention.

## Claims

1. An image processing apparatus (56) for performing a density correcting process on a radiographic image acquired using a recording plate (52) that is capable of storing radiation information, wherein:
the radiographic image includes a valid image region (72) corresponding to a region in which the recording plate (52) is present, and an invalid image region (74) corresponding to a region in which the recording plate (52) is not present;
the image processing apparatus (56) comprising:
a correction target region identifying unit (82) for identifying a region (94) in a predetermined range of the valid image region (72), which is adjacent to the invalid image region (74), as a correction target region (90); and
a density correction unit (84, 120) for removing a trend of density values of the correction target region (90), which is present from the valid image region (72) toward the invalid image region (74), thereby correcting the density values of pixels in the correction target region (90).

2. An image processing apparatus (56) for performing a density correcting process on a radiographic image acquired using a solid-state detector (52), wherein:
the radiographic image includes a valid image region (72) corresponding to a region that is irradiated with radiation, and an invalid image region (74) corresponding to a region that is not irradiated with radiation;
the image processing apparatus (56) comprising:
a correction target region identifying unit (82) for identifying a region (94) in a predetermined range of the valid image region (72), which is adjacent to the invalid image region (74), as a correction target region (90); and
a density correction unit (84, 120) for removing a trend of density values of the correction target region (90), which is present from the valid image region (72) toward the invalid image region (74), thereby correcting the density values of pixels in the correction target region (90).

3. The image processing apparatus (56) according to claim 1, wherein the density correction unit (84) corrects the density values of pixels in the correction target region (90) by increasing density values to be added to pixels in the correction target region (90) as the pixels are closer to the invalid image region (74).

4. The image processing apparatus (56) according to claim 1, wherein within the valid image region (72), a region other than the correction target region (90) is regarded as a region of interest (96); and
the density correction unit (84) includes a map calculator (100) for calculating an additive quantity map representing additive quantities to be added to the density values of a plurality of pixels in the correction target region (90) by calculating differences between the density values of a plurality of reference pixels serving as a reference for calculating the additive quantity map in the region of interest (96) and the density values of the pixels in the correction target region (90), a low-pass filtering processor (102) for performing a low-pass filtering process on the additive quantity map, and a density value adder (104) for adding the additive quantity map, which has been processed by the low-pass filtering process, to the density values of the pixels in the correction target region (90), so that the density correction unit (84) removes the trend of density values of the correction target region (90).

5. The image processing apparatus (56) according to claim 1, wherein within the valid image region (72), a region other than the correction target region (90) is regarded as a region of interest (96); and
the density correction unit (84) includes a low-pass filtering processor (102) for performing a low-pass filtering process on the density values of a plurality of pixels in the correction target region (90), a map calculator (100) for calculating an additive quantity map representing additive quantities to be added to the density values of the pixels in the correction target region (90) by calculating differences between the density values of a plurality of reference pixels serving as a reference for calculating the additive quantity map in the region of interest (96) and the density values of the pixels in the correction target region (90), which have been processed by the low-pass filtering process, and a density value adder (104) for adding the additive quantity map to the density values of the pixels in the correction target region (90), so that the density correction unit (84) removes the trend of density values of the correction target region (90).

6. The image processing apparatus (56) according to claim 4, wherein the map calculator (100) calculates the additive quantity map by calculating differences between the density values of the pixels in the correction target region (90) and the density values of the reference pixels that are shortest in distance from the pixels in the correction target region (90).

7. The image processing apparatus (56) according to claim 1, wherein within the valid image region (72), a region other than the correction target region (90) is regarded as a region of interest (96); and
the density correction unit (120) includes a low-pass filtering processor (122) for performing a low-pass filtering process on the density values of a plurality of pixels in the correction target region (90), a differential density value calculator (124) for calculating differential density values of the pixels in the correction target region (90) by calculating differences between the density values of the pixels in the correction target region (90) and the density values of the pixels in the correction target region (90), which have been processed by the low-pass filtering process, and an adder (126) for adding the density values of a plurality of reference pixels serving as a reference in the region of interest (96) to the differential density values of the pixels in the correction target region (90), so that the density correction unit (120) corrects the density values of the pixels in the correction target region (90).

8. The image processing apparatus (56) according to claim 7, wherein the adder (126) adds the pixels in the correction target region (90) and the reference pixels that are shortest in distance from the pixels in the correction target region (90).

9. The image processing apparatus (56) according to claim 4, wherein a boundary line (98) between the correction target region (90) and the region of interest (96) and a boundary line (92) between the valid image region (72) and the invalid image region (74) lie parallel to each other.

10. The image processing apparatus (56) according to claim 4, wherein the reference pixels comprise pixels in the region of interest (96) that are adjacent to the pixels in the correction target region (90).

11. The image processing apparatus (56) according to claim 1, wherein the correction target region identifying unit (82) identifies, as the correction target region (90), a region in the valid image region (72) that lies within a constant distance from a boundary line (92) between the valid image region (72) and the invalid image region (74).

12. The image processing apparatus (56) according to claim 1, wherein the correction target region identifying unit (82) identifies, as the correction target region (90), the invalid image region (74) together with the region (94) in the predetermined range of the valid image region (72) that is adjacent to the invalid image region (74).

13. The image processing apparatus (56) according to claim 1, wherein the density correction unit (84, 120) blackens out the invalid image region (74) by replacing the density values of pixels in the invalid image region (74) with a predetermined density value.
